# EUROPEAN PATENT APPLICATION

(11) **EP 1 207 209 A2**
(43) Date of publication of application: **22.05.2002**
(21) Application number: 01123992.8
(22) Date of filing: 08.10.2001
(51) Int. Cl.: C12Q 1/68

(54) **Methods using arrays for detection of single nucleotide polymorphisms**

(30) Priority: 09.11.2000 US 710983
(71) Applicant: Agilent Technologies Inc. (a Delaware Corporation), Palo Alto, CA 94306-2024 (US)
(72) Inventor: Amorese, Douglas A., Los Altos, California 94022 (US); Sampson, Jeffrey R., Burlingame, California 94010 (US)
(74) Representative: Schoppe, Fritz, Dipl.-Ing.

(57) **Abstract**

A method of evaluating for the presence of a single nucleotide polymorphism in a target (9). The method uses an addressable array (2) of probes (8) linked to a substrate (1). An analyte is exposed to the array (2) and a set of probes (8), such that the target (9) that may be present will bind to the probes (8). After the target (9) has hybridized to the probe (8), the probe (8) is extended using a DNA polymerase (13). A labeled (12) RAPD primer is used to produce a cDNA to the extended probe (8). Thermocycling is then used to amplify the labeled (12) products produced. A binding pattern on the array (2) is observed and the presence of the target (9) evaluated based on the binding pattern. Kits for detecting single nucleotide polymorphisms using these arrays (2) are also disclosed.

## Description

### FIELD OF THE INVENTION

This invention relates to arrays, and more particularly to the use of arrays to detect single nucleotide polymorphic arrays (SNP's), which are useful in diagnostic, screening, gene expression analysis, and other applications.

### BACKGROUND OF THE INVENTION

Various arrays of peptides and polynucleotides (such as RNA and DNA) are known and used in genetic testing, screening and diagnostics. Arrays are defined by the regions of different biopolymers or nucleotides arranged in a predetermined configuration on a substrate. Most importantly, the arrays when exposed to a population of analytes will exhibit a pattern indicative of the presence of the various components separated spatially. Array binding patterns of polynucleotides and/or peptides can be detected by using a variety of suitable fluorescent target labels. Once bound to the array, these target labels can then be quantified and observed and the overall fluorescence pattern on the array determined.

DNA microarrays are particularly useful for analyzing large sets of genes through "gene expression profiling". Using various techniques, arrays can be used to effectively analyze genomes and portions of genomes. Probe arrays have been produced by a variety of means. However, two major methods exist for fabricating arrays used in expression profiling. The first technique uses chemical methods to synthesize polynucleotide probes *in situ* on array surfaces. This technique uses addressable adaptions of phorsphoramidite chemistry. In the second method, polynucleotide probes synthesized enzymatically or chemically can be deposited and attached to a surface through covalent or non-covalent means. The enzymatic method is particularly effective in fabricating arrays with larger probes of (100-1000 nucleotides).

Shorter synthetic probes provide improved specificity and discrimination over longer probe types, because of their ability to distinguish between sequences that are closely related. However, these same probes suffer from the disadvantage that there are less Watson-Crick base pairs and weaker association of probe and target molecules making for reduced binding affinity.

Of particular value are scientific advances that pinpoint individual variations in the human genome (polymorphisms). Polymorphisms are important because they may be routinely detected and applicable in a clinical setting. Polymorphisms can be used as diagnostic or prognostic parameters in disease, potential predictors of drug response in a patient as well as providing a variety of information for future drug design. The best understood polymorphisms in humans are the single nucleotide polymorphisms (SNPs). SNPs can be linked directly or indirectly to an alteration within the coding sequence of a gene that may determine an individual's response to a particular drug. The ability to identify and classify various individuals according to genetic background as well as SNP profile is a powerful way for customizing the drug design process. This makes SNPs an important area for research.

The idea of using SNP analysis on DNA micro-arrays is fairly new. Such a technique promises to be accurate and simple compared to other techniques in determining important genes and polymorphisms of a defined genome. Typically, SNP analysis on DNA micro-arrays requires the amplification of the target genome in order to reduce the complexity of the sample and have sufficient signal for detection. Thousands of distinct regions need to be amplified in order to survey an entire genome. However, a barrier to designing a genome wide SNP array based assay, is the complexity of multiplexing the nucleic acid amplification method, polymerase chain reaction (PCR), to generate thousands of fragments. For instance, as the number of primers increase, the potential for non-specific priming, or the segment failing to amplify, increases exponentially. In addition, there is also a cost and complexity factor in synthesizing a large number of different primers. Currently, techniques exist to do SNP analysis on limited regions known to be of interest or to gang multiple multiplex PCRs. A brief review of these techniques is, therefore, necessary to best understand the present invention.

The typical techniques rely upon knowledge of a gene, protein, or other specific sequence known *a priori* to be highly conserved throughout a specific genome. Since restriction enzymes digest DNA at specific sequences, a point mutation within this site results in the loss or gain of a recognition site, giving rise in that region to restriction fragments of different length. Mutations caused by the insertion, deletion or inversion of DNA stretches will also lead to a length variation of DNA restriction fragments. Genomic restriction fragments of different lengths between genotypes can be detected on Southern blots (Southern, J. Mol. Biol. 98, 503, (1975)). The genomic DNA is typically digested with any restriction enzyme of choice, the fragments are electrophoretically separated, and then hybridized against a suitably labeled probe for detection. The sequence variation detected by this method is known as restriction length polymorphism or RFLP (Botstein et al., Am. J. Hum. Genet. 342, 314, (1980)). RFLP genetic markers are particularly useful in detecting genetic variations in phenotypically silent mutations and serve as highly accurate diagnostic tools.

Nucleotide mutations or polymorphisms have also been detected using amplification schemes. Such methods include the "polymerase chain reaction" (PCR)(Mullis et al., United States Patent No., 4,683,195) and a number of transcription-based amplification methods (Malek et al., U.S. Patent No. 5,130,238; Kacian and Fultz, U.S. Patent No. 5,399,491; Burg et al., U.S. Patent No. 5,437,990). PCR type reactions are particularly useful. For example, polymerase chain reaction (PCR) or ligase chain reaction (LCR) have been used to amplify particular strands of DNA of known sequence and with defined primers. If PCR methodology is selected, the amplification method would include a replication composition consisting of, for example, nucleotide triphosphates, two primers of specific sequence and, DNA or RNA polymerases. These reagents and details describing procedures for their use in amplifying nucleic acids are provided in U.S. Pat. No. 4,683,202 (1987, Mullis et al.) and U.S. Pat. No. 4,683,195 (1986, Mullis et al.). However, PCR and each of the above described amplification schemes requires at least two primers of a specific sequence. In addition, these primers must be complementary to different strands of a desired nucleic acid sequence and result in an exponential increase in the number of copies of the target sequence. These methods, however, have limitations in that dual primers must be used and sequences must be known of both the primer and sequence or region to be amplified. These limitations, therefore, make the sole use of these techniques limited in gene expression monitoring applications.

Other methods have been developed for identifying genetic polymorphic markers. For instance, these techniques employ DNA amplification using short primers of arbitrary sequence. These primers have been termed "random amplified polymorphic DNA" or "RAPD" primers (see Williams et al., Nucl. Acids. Res., 18, 6531 (1990) and U.S. Pat. No. 5,126,239; also EP 0 543 484 A2, WO 92/07095, WO 92/07948, WO 92/14844, and WO 92/03567). The RAPD method amplifies either double or single stranded non-targeted, arbitrary DNA sequences using standard amplification buffers, deoxyribonucleotide triphosphates and a thermostable DNA polymerase such as Taq. The nucleotide sequence of the primers is typically about 9 to 13 bases in length, between 50 and 80% G+C in composition and contains no palindromic sequences. *RAPD* detection of genetic polymorphisms represents an advance over RFLP in that it is less time consuming, more informative, and readily susceptible to automation. RAPD also has advantages over PCR and other similar amplification schemes because of the ability to amplify a number of defined areas on a DNA strand with only the need of a single primer. In addition, primers and genetic sequences may be random or unknown. Because of its sensitivity for the detection of polymorphisms, *RAPD* analysis and variations based on RAPD/PCR methods have become the methods of choice for analyzing genetic variation within species or closely related genera, both in the animal and plant kingdoms.

These references, and all other references cited in this application, are incorporated in this application by reference. However, cited references or art are not admitted to be prior art to this application.

It would be desirable then, to provide a means for detecting a target or target sequence using probes, particularly in the form of an addressable SNP array, which can provide good binding affinity and specificity for a target. It would also be desirable to combine the strengths of the above PCR and RAPD techniques to construct an array based system or methodology for SNP analysis that is rapid, efficient and sufficiently sensitive to avoid the need for multiplex PCR amplification of genomic regions to be analyzed.

### SUMMARY OF THE INVENTION

The invention is a method and kit for detection of single nucleotide polymorphisms in a target. The target may be genomic DNA, oligonucleotides or polynucleotides. The invention includes evaluating the presence of the target in an analyte using an addressable array of probes linked to a substrate. The probes may be nucleotides, oligonucleotides or polynucleotides. The steps in the detection process include exposing the analyte to the array and a set of linked probes such that the target that may be present will hybridize to the probes. Next, the probes are extended by at least two nucleotides using a DNA polymerase. The observed binding pattern can then be observed on the array and evaluated for the presence of the target. The binding pattern need not be observed immediately after the probes have been extended. A number of labeled polynucleotide primers may also be added and the primers extended by the DNA polymerase back toward the array surface. Thermocycling can then be used to amplify the extended labeled products.

The invention also includes a kit for detection of single nucleotide polymorphisms in a target. The kit comprises an addressable array having probes, a DNA polymerase for extending the probes and a labeled primer for synthesizing cDNA to the extended probes.

### BRIEF DESCRIPTION OF THE DRAWINGS

Embodiments of the invention will now be described with reference to the drawings in which:
FIG. 1 illustrates a single nucleotide polymorphic array, at least one of which is of the present invention, wherein the probes are directly linked to the substrate;
FIG. 2 is an enlarge view of a portion of FIG. 1 showing multiple spots or regions of one array;
FIG. 3A illustrates the first step in the SNP detection scheme.
FIG. 3B illustrates the second step in the SNP detection scheme.
FIG. 3C illustrates the third step in the SNP detection scheme.
FIG. 3D illustrates the fourth step in the SNP detection scheme.
FIG. 3E illustrates the fifth step in the SNP detection scheme.
FIG. 4 illustrates an array of the present invention showing how a series of features might be used to determine differences in genotypes.

### DETAILED DESCRIPTION OF THE INVENTION

Before describing the present invention in detail, it is to be understood that this invention is not limited to specific compositions, process steps, or equipment, as such may vary. It is also to be understood that the terminology used herein is for the purpose of describing particular embodiments only, and is not intended to be limiting.

It must be noted that, as used in this specification and the appended claims, the singular forms "a", "an" and "the" include plural referents unless the context clearly dictates otherwise. Thus, for example, reference to "an array" includes more than one array, reference to "a polynucleotide primer" includes a plurality of polynucleotide primers and the like.

In describing and claiming the present invention, the following terminology will be used in accordance with the definitions set out below.

A "biopolymer" is a polymer of one or more types of repeating units. Biopolymers are found in biological systems and particularly include peptides and polynucleotides, as well as such compounds composed of or containing amino acid or nucleotide analogs or non-nucleotide groups. This includes polynucleotides in which the conventional backbone has been replaced with a non-naturally occurring or synthetic backbone, and nucleic acids in which one or more of the conventional bases have been replaced with a synthetic base capable of participating in Watson-Crick type hydrogen bonding interactions. Polynucleotides include single or multiple stranded configurations, where one or more of the strands may or may not be completely aligned with another. While probes and targets of the present invention will typically be single-stranded, this is not essential. Specifically, a "biopolymer" includes DNA (including cDNA), RNA and polynucleotides, regardless of the source.

A "nucleotide" refers to a sub-unit of a nucleic acid and has a phosphate group, a 5-carbon sugar and a nitrogen containing base, as well as analogs of such sub-units. An "oligonucleotide" generally refers to a nucleotide multimer of about 10 to 100 nucleotides in length, while a "polynucleotide" includes a nucleotide multimer having any number of nucleotides. A "biomonomer" references a single unit, which can be linked with the same or other biomonomers to form a biopolymer (for example, a single amino acid or nucleotide with two linking groups one or both of which may have removable protecting groups). A biomonomer fluid or biopolymer fluid reference a liquid containing either a biomonomer or biopolymer, respectively (typically in solution).

An "array", unless a contrary intention appears, includes any one or two dimensional arrangement(s) of addressable regions bearing particular biopolymer moieties (for example different polynucleotide sequences) associated with that region. An array is "addressable" in that it has multiple regions of different moieties (for example, different sequences) such that a region at a predetermined location (an "address") on the array (a "feature" of the array) will detect a particular target or class of targets (although a feature may incidentally detect non-targets of the feature). In the present case, the polynucleotide (or other) target will be in a mobile phase (typically fluid), while probes for the target ("probes") may or may not be mobile (as described in this application). "Hybridizing" and "binding", with respect to polynucleotides, are used interchangeably. "Binding efficiency" refers to the productivity of a binding reaction, measured as either the absolute or relative yield of binding product formed under a given set of conditions in a given amount of time. "Hybridization efficiency" is a particular sub-class of binding efficiency, and refers to binding efficiency in the case where the binding components are polynucleotides. It will also be appreciated that throughout the present application, that words such as "upper", "lower" are used in a relative sense only. A "set" may have one type of member or multiple different types. "Fluid" is used herein to reference to a liquid.

The term "target" shall refer to a nucleic acid, nucleotide, nucleoside or their analogs. The term shall also include nucleotides having modified sugars as well as organic and inorganic leaving groups attached to the purine or pyrimidine rings.

The term "probe" shall refer to a nucleic acid, nucleotide, nucleoside or their analogs. The term shall also include nucleotides having modified sugars as well as organic and inorganic leaving groups attached to the purine or pyrimidine rings.

The term "primer-directed amplification" refers to any of a number of methods known in the art that result in amplification of nucleic acid molecules using the recognition of a specific nucleic acid sequence or sequences to initiate an amplification process. Applicants contemplate that amplification may be accomplished by any of several schemes known in this art, including but not limited to the polymerase chain reaction (PCR) or ligase chain reaction (LCR). If PCR methodology is selected, the amplification method would include a replication composition consisting of, for example, nucleotide triphosphates, two primers with appropriate sequences, DNA or RNA polymerases and proteins. These reagents and details describing procedures for their use in amplifying nucleic acids are provided in U.S. Pat. No. 4,683,202 (1987, Mullis et al.) and U.S. Pat. No. 4,683,195 (1986, Mullis et al.).

The term "primer" refers to a nucleic acid fragment or sequence that is complementary to at least one section along a strand of the sample nucleic acid, wherein the purpose of the primer is to sponsor and direct nucleic acid replication of a portion of the sample nucleic acid along that string. Primers can be designed to be complementary to specific segments of a targeted sequence. In PCR, for example, each primer is used in combination with another primer forming a "primer set" or "primer pair"; this pair flanks the targeted sequence to be amplified. In *RAPD* amplification, single arbitrary primers are used to amplify non-targeted segments of nucleic acids that are located between the primer sequence sites in opposing DNA strands. The term "primer", as such, is used generally herein to encompass any sequence-binding polynucleotide which functions to initiate the nucleic acid replication process. The term also includes nucleotide modifications that would be present or used in the primer itself. These type modifications would be for the purpose of increasing hybridization stability, specificity and reducing unwanted intramolecular structures.

The term "amplification product" refers to specific DNA fragments generated from any primer-directed nucleic acid amplification reaction.

The term "derived from", with reference to an amplification primer, refers to the fact that the sequence of the primer is a fragment of the sequence from which it has been "derived". The fragment is always denoted in a 5' to 3' orientation. The useful primer sequence size range for PCR amplification is about 15 base pairs to about 30 base pairs in length.

The term *"RAPD"* refers to random amplified polymorphic DNA. *"RAPD* amplification" refers to a method of single primer-directed amplification of nucleic acids using short primers of arbitrary sequence to amplify non-targeted, random segments of nucleic acid. The method is disclosed and claimed in U.S. Pat. No. 5,126,239. *"RAPD* method" or "*RAPD* analysis" refers to a method for the detection of genetic polymorphisms involving the non-targeted amplification of nucleic acids using short primers of arbitrary sequence, whereby the profile or pattern of *RAPD* amplification products is compared between samples to detect polymorphisms. *"RAPD* primers" refers to primers of about 8 to 13 bp, of arbitrary sequence, useful in the *RAPD* amplification or *RAPD* analysis according to the above method.

When one item is indicated as being "remote" from another, this is referenced that the two items are at least in different buildings, and may be at least one mile, ten miles, or at least one hundred miles apart. "Communicating" information references transmitting the data representing that information as electrical signals over a suitable communication channel (for example, a private or public network). "Forwarding" an item refers to any means of getting the item from one location to the next, whether by physically transporting that item or otherwise (where that is possible) and includes, at least in the case of data, physically transporting a medium carrying the data or communicating the data.

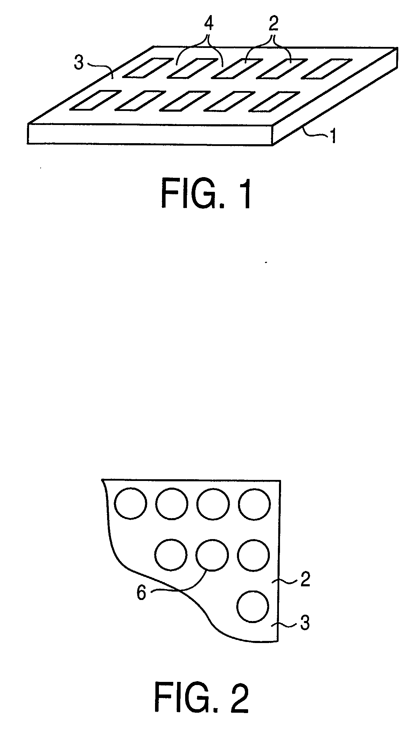

Referring first to FIGS. 1-3, typically kits and methods of the present invention use a contiguous substrate 1 carrying arrays 2 disposed across an array surface 3 of substrate 1 and separated by areas 4. The arrays on substrate 1 can be designed for testing an analyte or for evaluating probes on their ability to form hybrids. While a number of arrays 2 are displayed and shown in FIG. 1, the different embodiments described below may use substrates with particular numbers of arrays, it will be understood that substrate 1 and the embodiments to be used with it may use any number of desired arrays 2. Similarly, substrate 1 may be of any shape, and any apparatus used with it adapted accordingly. Depending upon intended use, any or all of arrays 2 may be the same or different form one another and each will contain multiple spots or features 6 of biopolymers in the form of polynucleotides. A typical array may contain from 100 to 100,000 regions. All of the features 6 may be different, or some or all could be the same. Each feature carries a predetermined polynucleotide having a particular sequence, or a predetermined mixture of polynucleotides. It will be appreciated though, that there need not be any space separating arrays 2 from one another, no features 6 within an array from one another.

The invention includes the use of the array 2 for SNP analysis, where the array probes 8 are 5' attached with the 3' hydroxyl end free in solution. The array probes 8, are configured on array surface 3 spatially separated into distinct features 6A and reflect the sequence flanking the SNP where the last nucleotide in the polynucleotide corresponds (or is complementary) to the polymorphic base in the target 9. Target 9 may include a variety of nucleotide, polynucleotide or genomic DNA and/or RNA. The genome to be analyzed or target 9 is distributed across the array 2 in a thermophilic polymerase containing dNTPs and a limited set of short non-specific fluorescently labeled polynucleotide primers 11(9 to 15 oligomers). These polynucleotide primers 11 may be labeled with a variety of different types of labels including radioisotopes, chemiluminescent probes as well as fluorophores and chromophores 12. The polynucleotide primers 11 are chosen such that statistically one of them should fall within a defined distance (i.e. 1kb) of any given position within a genome. The solution in contact with the array 2 is contained in a vapor tight fixture and heat cycled from about 95 °C to about 70 °C for about 25 to 35 cycles. During this process, the target 9 is denatured and allowed to anneal specifically to the probes 8 on array surface 3. If the 3' terminal nucleotide of the surface bound probe 8 is complementary to the nucleotide in the target 9, the thermophilic DNA polymerase 13 will extend the probe 8 for several bases (generally greater than 1kb) to form probe 8'. Following denaturation, the surface bound extended probe 8'is primed with one of the short fluorescently labeled polynucleotide primers 11 and extended back toward the array surface 3 of array 2 with the DNA polymerase 13, creating a fluorescent dsDNA molecule attached at the array surface 3, where the polynucleotide primer 11, complementary to the probe 8' was present. Denaturation of this complex results in a new target molecule 11' in solution for hybridization to a second immobilized polynucleotide (note: the extension product of polynucleotide primer 11 is the new target molecule 11'). In this way the SNPs can be detected without amplifying the target 9 in a multiplex PCR reaction. Potentially, thousands of PCR reactions can take place in distinct areas of the array 2. Signal should be amplified exponentially, but it is anticipated that the reaction will plateau as the effective concentration of synthesized fragments grow. Following the last extension reaction, the array 2 is washed under moderate to high stringency to remove any fluorescent primers or extension products that are not specifically bound. Presence of the fluorescent signal at any given location suggests the presence of the sequence within the target 9, specifically one matching the probe 8 at its 3' end. By having 2 or 4 related polynucleotides differing only by their terminal 3' base, one can determine whether the target 9 is heterozygous or homozygous for a specific SNP.

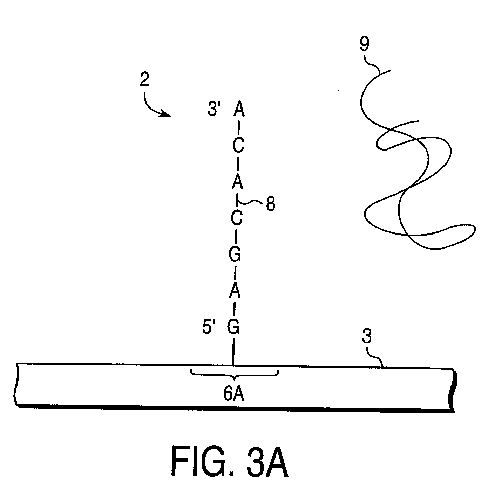

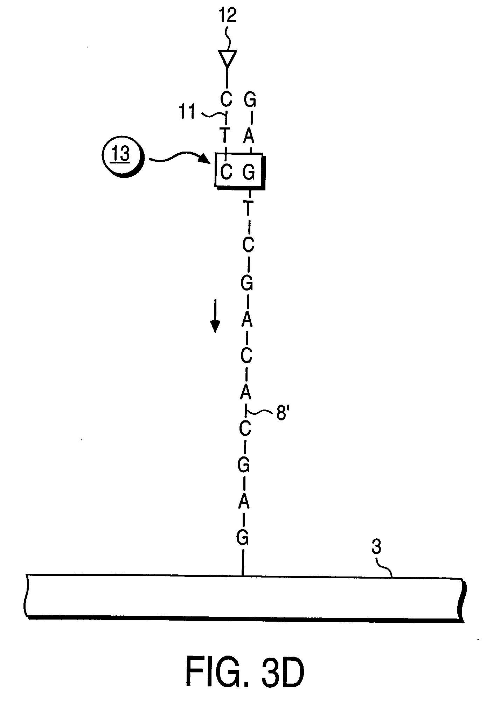

FIGS. 3A-3E show the detailed steps of the present invention and how the array(s) 2 may be used to detect SNPs in genomic DNA.

FIG. 3A shows the array 2 with bound probe 8 on the array surface 3. The probe 8 is attached using the 5' end of the nucleotide using phosporamidite chemistry or similar techniques know in the art. The important feature is that the 3' hydroxyl end is free in solution. This allows for hybridization to target 9 and extension of the probe 8. The importance of the primary sequence of the probe 8 is that it allows hybridization or annealing to important regions in the target 9 (genomic DNA) that contains the SNP that is under study. Probe 8 may be of any determined or undetermined sequence and will be discussed in more detail below. Probe 8 may comprise a variety of sizes for interaction with the target 9. For clarity, the diagram 3A shows the probe 8 having only 7 nucleotide bases. In its preferred embodiment the probe 8 is probably a biopolymer of around 10-30 mers and more preferably around 25 mers in length. Array feature(s) 6 or 6A, therefore, may be designed to include a variety of similar sequenced probes 8. Array features 6, may be defined to include over 2000 similarly sequenced and attached probes 8 all capable of binding and probing target 9.

Target 9, as discussed, may include a variety of nucleic acids, polynucleotides or DNA. In particular, target 9 may be genomic DNA that includes both eucaryotic and prokaryotic nucleic acids that may be of interest or that contain SNPs for analysis. For instance, human DNA is known to contain at least 3 billion nucleotides with approximately 1 in every 1000 of those nucleotides being a SNP. Therefore, there are approximately 3 million SNPS that may be of interest in characterizing and analyzing in the complete genomic DNA. It should be noted that in its preferred embodiment, target 9, is genomic DNA and more particular random fragments of around 1 kb that have been produced by truncating or chopping up the original genomic DNA. In addition, the genomic DNA includes both the sex chromosomal DNA and the somatic cell DNA. In other words, there is no need to separate the somatic cell DNA from the sex chromosome DNA when using the present invention. Simply, the entire DNA needs to be isolated and then subject to either enzymatic or mechanical means that will shorten the stands. Enzymatic means may include hydrolysis reactions as well as digestion of the genomic DNA by means of restriction endonucleases. Mechanical means may include subjecting the DNA to shearing forces, ultrasonic waves, microwaves, heating or other similar methods known in the art. The genomic DNA is then simply exposed in solution to the probe 8 for binding or annealing.

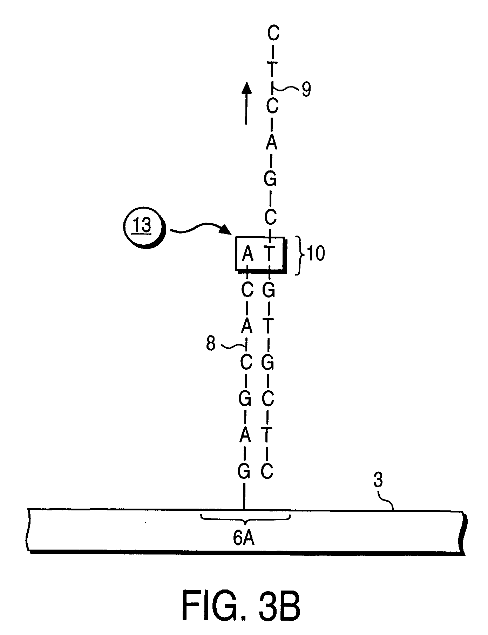

FIG. 3B shows the second step in the method using the array 2. The target 9 has been split into smaller fragments. For convenience, FIG. 3B shows target 9 as a 13 nucleotide base fragment. Of particular importance, is the fact that the seventh base contains a SNP. In the diagram the seventh base is boxed and marked as reference numeral 10. Position 7 is the terminal nucleotide of the probe 8. However, if probe 8 is a 25 mer the terminal base will be position 25. The important point being that the final nucleotide of probe 8 can be engineered synthetically or otherwise to be any of the pyrimidine or purine bases. Complementarity of bases allows for determining the SNP in the genomic DNA. In addition, from time to time alterations may exist in the DNA of various individuals and groups in the defined feature 6A (i.e. the position 7 or position 25 etc.). By separately synthesizing the probe 8 with different terminal bases, comparisons can be made with variations in the bases and positions to determine if the SNP is significant among a variety of genomic DNA or is just individually specific to the genomic DNA under study. This is explained in more detail below.

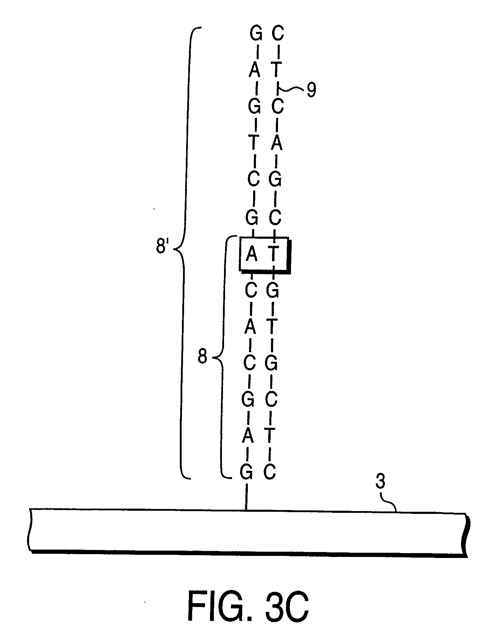

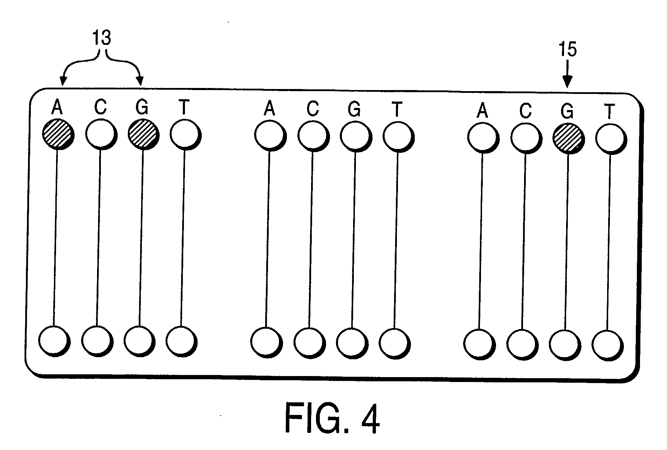

FIG. 3B shows the target 9 hybridized to the probe 8. Annealing takes place by lowering the temperature of the system when complementary base pairing strands are in close proximity. If position 7 of the probe 8 is an adenine, the corresponding base in the hybridized target 9, must be a thymine for the polymerase 13 to extend probe 8 in the direction of the arrow shown in the FIG. 3B. FIG. 3C shows the results when the target 9 has hybridized and both the target 9 and polynucleotide 8 contain matching bases. The probe 8 will be extended by a number of nucleotides by the polymerase 13. In particular, the polymerase 13 will generally extend the probe 8 by around 1.5 kilo bases (kb). For convenience, the drawing shows probe 8 extended by only six bases. As discussed, if position 7 of probe 8 is guanine (G), thymine (T), or cytosine (C), the polymerase 13 will not extend the probe 8 to make probe 8', because while there may be regions of base pairing, there is no base pairing at the strands terminal base of probe 8 and target 9. This is an important feature of the invention since the other arrays features 6 that might be constructed with G, T, or C in position 7 (a the terminal end of probe 8) will not be extended (See FIG. 4). In other words, probe 8 with the adenine (A) in position 7 will be easily distinguished from the other array probe(s) that will not be extended. After probe 8 has been extended to match the target 9 strand and form probe 8', the system is subject to a heat cycle that goes from 70 °C to about 95°C (i.e. standard PCR cycling) to allow for the separation and removal of the target 9 from the extended probe 8'.

The temperature of the system is then again lowered and the surface bound probe 8' can then be primed by one of a number of separately labeled polynucleotide primers 11 that are already present in solution. The polynucleotide primer 11 may comprise one or more labels 12 that may provide a means for measuring the levels of extended DNA present in the array 2. Polynucleotide primer 11 may be labeled using fluorescent, radioisotope, or chemiluminescent technology. The labeled polynucleotide pimer 11 may be a random or non-random primer and generally range in size of up to 15 kb. The polynucleotide primers 11 are chosen such that statistically one of them should fall within a defined distance (i.e. 1 kb) of any given position within a defined genome. In other words typical RAPD primers are employed at this step in the invention. Primer directed amplification is then employed. For instance, once the appropriate polynucleotide primer 11 has hybridized to the extended probe 8', the polynucleotide primer 11 is extended back toward the array surface 3 by DNA polymerase 13 for several bases (generally until the point of attachment to the surface). The RAPD amplification product or amplified product of the RAPD methodology is the polynucleotide 11'.

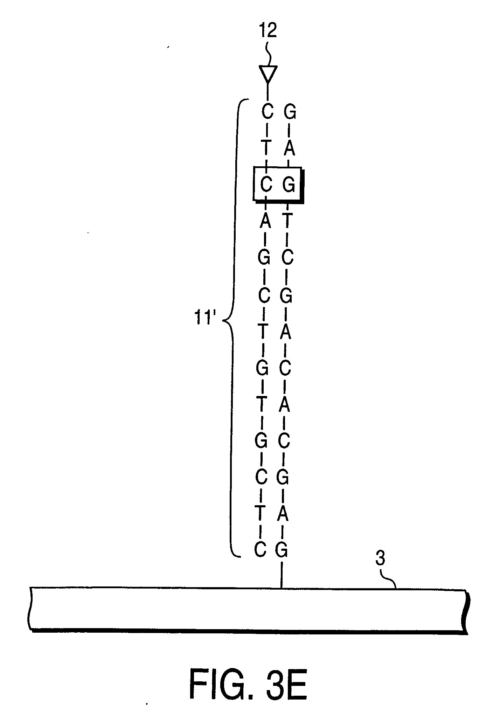

FIG. 3E shows the results of the extension of the oligomer primer 11 back to the array surface 3. Denaturation of this complex then results in a new polynucleotide 11' in solution for hybridization to a second immobilized probe 8'. Amplification and further production of products at this step in the methodology will generally follow standard PCR methodology. The above RAPD and PCR amplifcation strategies maintain a high amount of binding efficiency or hybridization efficiency due to the size of primers employed and the length of the amplified products that are produced.

FIG. 4 shows a plan view of the array 2 having features 6. Features 6 have been magnified for ease of viewing and are not drawn to scale. Each of the features 6 is represented by a circle. The letters above the circle(s) represent the terminal nucleotide present in a series of probe 8 that have been attached to substrate 1 within the feature 6 domain. For instance, "A" indicates that adenine is the terminal 3' base of a collection of polynucleotide within the feature 6, C represent cytosine etc. Each of the features 6 can be designed so that various segments of modified genomic DNA can be analyzed using the arrays 2 present on the substrate 1 (as shown in FIG. 4). A series of features 6 can be used for typing the actual SNP polymorphisms that may exist in the genomic DNA under study. In particular, the arrays can be designed for actually typing genomic DNA for particular allele traits. For instance, it can be determined whether a particular individual is homozygous or heterozygous for a particular trait where there is fluorescence. Features 6 can be organized in a particular fashion on the array surface 3 so that various sequential systems can be employed on the array 2. For instance, FIG. 4 shows the case scenario where A, C, G and T are the terminal base in the probe 8 that is used to probe the target 9. A second set of features 6 in a similar order A, C, G, and T can then be used to probe the next 7 or 25 nucleotides in the genomic DNA under study. This can be repeated and the patterns determined for the whole genomic DNA.

Reference number 13 of FIG. 4 shows the case scenario when an individual may be heterozygous for a particular trait. The feature 6 shows both the "A" and the "G" exhibit fluorescence patterns. Reference numeral 15 shows the case scenario when the individual is homozygous for a particular trait. Only one feature 6 or the "G" shows fluorescence. Since the probed genomic DNA includes both the sex and somatic cell DNA, both parental alleles for the trait may be present. Differences in polymorphisms must be compared in other organisms (i.e. to a human population) to determine if the actual features 6 that fluoresce are consistent or significant polymorphisms among a population. Consistent array patterns for a population indicate important SNPs or alleles.

### EXAMPLE 1

A specific example of the process of selecting a SNP hybridizing probe set, will now be described.

### Selection of successful individually hybridizing candidate probes:

Highly sensitive probe sequences will be determined by two-step iterative refinement. In the first, every 10^{th} possible 25-mer probe to each target will be synthesized on an polynucleotide array. Arrays will be hybridized to rhodamine-6-G-labeled complementary RNA (cRNA) derived from each target..

### Array construction and SNP analysis:

5' to 3' nucleotide arrays will be produced by deposition of polynucleotides on an amine surface. Initial targets will be selected from a set of yeast genes where sequences are well known and studied and which contained well defined polymorphic regions. A reaction mixture containing: yeast DNA, Taq polymerase, PCR buffer, nucleotides and a set of 10 cyanine labeled polynucleotides (12 nucleotide base polymers-serving as non-specific primers) will be employed . Reactions will be performed in modified chambers or seal-a-meal bags or in an "air cycler" . First phase experimentation will focus on identifying DNA polymerases that worked effectively at the above described surfaces and do not extend off mismatched 3' ends. Important to the experiment will be the use/composition of tethers for application with surface bound polynucleotides to solution, temperatures/time/number of cycles.

SNPs will be detected without amplifying the target in a multiplex PCR reaction. Following the last extension reaction the arrays will be washed under moderate to high stringency conditions to remove any fluorescent primers or extension product that will not specifically bind. A confocal scanner will be used to determine the presence of fluorescent signal at any defined location or area. By having 2 or 4 related polynucleotides differing only by their 3' base, it will be easy to determine whether the target molecules are heterozygous or homozygous.

When a user receives an array made by an apparatus or method of the present invention, it will typically be exposed to a sample and the array interrogated following exposure. Interrogation is usually accomplished by a suitable scanner that can read the location and intensity of fluorescence at each feature of an array following exposure to a fluorescently labeled sample (such as a polynucleotide containing sample). For example, such a scanner may be similar to the fluorescent scanner available from Agilent, Inc., Palo Alto, CA. Results from the interrogation can be processed results, such as obtained by rejecting a reading for a feature which is below a predetermined threshold and/or forming conclusions based on the pattern read from the array (such as whether or not a particular target sequence may have been present in the sample). The results of the interrogation (processed or not) can be forwarded (such as by communication) to a remote location if desired and received there for further use.

Various modifications to the embodiments of the invention described above are, of course, possible. Accordingly, the present invention is not limited to the particular embodiments described in detail above.

## Claims

1. A method of evaluating for the presence of a target (9) in an analyte, using an addressable array (2) of probes (8) linked to a substrate (1), comprising:
(a) preparing an analyte solution consisting of a buffer, target nucleic acid (9), DNA polymerase (13), deoxynucleotides, and polynucleotide primers (11);
(b) exposing the analyte solution to the array (2) and the set of probes (8), such that the target (9) that may be present will hybridize to at least one of the probes (8);
(c) extending the probes (8) that have been bound to the target (9); and
(d) observing a binding pattern on the array (2) and evaluating for the presence of the target (9) based on the observed binding pattern.

2. A method according to claim 1, wherein the target (9) is an oligonucleotide or polynucleotide.

3. A method according to claim 1, wherein the probe (8) is an oligonucleotide.or polynucleotide.

4. A method according to claim 3, wherein the probe (8) further comprises a predetermined feature (6) for binding a target (9).

5. A method according to claim 4, wherein the predetermined feature (6) of the probe (8) is designed such that the terminal base of the probe (8) corresponds or is complementary to the corresponding base on the target (9).

6. A method according to claim 5, wherein the complementary base of the target (9) polynucleotide is polymorphic.

7. The method of claim 1, wherein the probes (8) are extended at least two nucleotide bases.

8. The method of claim 7, wherein at least one of the added nucleotide bases to the probe (8) has a label (12).

9. The method of claim 1, wherein the probes (8) are extended a number of nucleotide bases to form a polynucleotide.

10. A method according to claim 1, wherein based on the observed binding pattern, the target (9) is determined to have been present in the sample.

11. A method of using an array (2) to conduct detection of a target (9), comprising:
(a) distributing a target (9) to be analyzed across the array (2) in a buffer containing dNTPs and a set of labeled (12) polynucleotide primers (11), the primers (11) being chosen such that statistically one of them should fall within a defined distance of any given position within the target (9);
(b) processing the target (9) by allowing it to denature and then hybridize to the polynucleotides on the array (2) surface such that when the 3' terminal nucleotide of the polynucleotide on the array (2) surface is complementary to the nucleotide in the target (9) polynucleotide, a DNA polymerase (13) will extend the polynucleotide;
(c) further processing the target (9) polynucleotide by denaturing the product of step (c) and priming the product with an oligomer that is extended toward the array (2) surface with the DNA polymerase (13) to form a nucleic acid complex; and
(d) denaturing the DNA complex of step (c) so that hybridization to a second polynucleotide may take place.

12. A method of using an array (2) to conduct detection of a target (9), comprising:
(a) forming an array (2) of probes (8) on an array (2) surface;
(b) distributing a target (9) across the array (2) surface;
(c) allowing the target (9) to hybridize to the probes (8);
(d) extending the probes (8) at least two nucleotide bases with a DNA polymerase (13);
(e) denaturing the product of step (d) and priming the product with a labeled (12) primer;
(f) extending the primer toward the array (2) surface with the DNA polymerase (13) to form a double standed DNA complex; and denaturing the DNA complex of step (f) so that hybridization to a second polynucleotide may take place.

13. A method of using a probe (8) to detect a target (9) molecule, comprising:
(a) allowing the target (9) molecule to hybridize to the probe (8);
(b) extending the probe (8) with a polymerase;
(c) denaturing the product of (b); and
(d) hybridizing a primer to a product of (c).

14. A method as recited in claim 13, wherein said primer is random or non-random sequence.

15. A method as recited in claim 13, further comprising the step of extending the primer of (d) back toward the array (2).

16. A method of using a probe (8) to detect a target (9) molecule, comprising the steps of:
(a) allowing the target (9) molecule to hybridize to the probe (8);
(b) extending the probe (8) with a polymerase;

17. A method as recited in claim 16, further comprising the step of denaturing the product of (b) to form two DNA strands.

18. A method as recited in claim 17, further comprising the step of hybridizing a primer to one of the DNA strands produced by denaturing the product of (b).

19. A method as recited in claim 18, wherein said primer is random or non-random in sequence.

20. A method as recited in claim 16, further comprising the step of extending the hybridized primer back toward the array (2).

21. A process for determination of at least one target (9) nucleotide at least one particular location within at least one polynucleotide sequence in a test sample, said target (9) nucleotide(s) constituting a 5' terminal nucleotide of a target (9) nucleotide sequence within said polynucleotide sequence(s), said process including the steps of:
(a) immobilizing one or more arrays (2) of identical oligonucleotide primers with a solid support defining one or more reaction zones wherein each of said oligonucleotide primers in a respective array (2) is capable of hybridizing with a corresponding target (9) nucleotide sequence;
(b) contacting the reaction zone with the test sample;
(c) hybridizing as least one of said oligonucleotide primers with the polynucleotide sequence to form a hybrid wherein the polynucleotide sequence extends in a 3' to 5' direction beyond the 3' terminal nucleotide of the at least one oligonucleotide primer;
(d) extending the at least one oligonucleotide primer of the hybrid beyond the 3'terminal nucleotide thereof in the 5' to 3' direction using the polynucleotide sequence as a template in the case when the 3' terminal nucleotide of the at least one oligonucleotide primer is complementary to the 5' terminal nucleotide of said target (9) nucleotide sequence, said extension effected in the presence of a polymerization agent and nucleotide precursors to form a duplex including an extended primer molecule;
(e) denaturing the duplex to free the polynucleotide sequence from the extended primer molecule;
(f) adding a second primer to hybridize to the extended primer sequence; and
(g) carrying out the steps (c)-(f) one or more times.

22. The process of claim 21, wherein the second primer has a random sequence or wherein the step of immobilizing in (a) is **characterized in that** a single array (2) of identical oligonucleotide primers is immobilized at a single reaction zone of the solid support.

23. The process of claim 21, further comprising extending the second primer.

24. A kit for detection of single nucleotide polymorphisms in a target (9), comprising:
(a) an addressable array (2) having probes (8);
(b) a DNA polymerase (13) for extending probes (8); and a labeled (12) primer for synthesizing DNA to an array (2) surface.
